# EUROPEAN PATENT SPECIFICATION

(11) **EP 3 882 163 B1**
(45) Date of publication and mention of the grant of the patent: **22.04.2026**
(21) Application number: 19885074.5
(22) Date of filing: 31.10.2019
(51) Int. Cl.: A61M 5/178, A61J 15/00, A61M 5/31

(54) **SUCTION ADAPTOR FOR HIGHLY VISCOUS ENTERAL NUTRIENT**
SAUGADAPTER FÜR HOCHVISKÖSE ENTERALNAHRUNG
ADAPTATEUR D'ASPIRATION POUR NUTRIMENT ENTÉRAL HAUTEMENT VISQUEUX

(30) Priority: 12.11.2018 JP 2018212140
(43) Date of publication of application: 22.09.2021
(73) Proprietor: Matsumoto, Masaki, Mie 511-0811 (JP)
(72) Inventor: Matsumoto, Masaki, Mie 511-0811 (JP)
(74) Representative: Kronthaler, Schmidt & Coll.
(86) International application number: PCT/JP2019/042738
(87) International publication number: WO 2020/100605

(56) References cited:
- WO-A1-2014/063112
- WO-A1-2014/143130
- JP-A- 2011 024 687
- JP-A- 2012 045 444
- JP-A- S6 333 202
- US-A- 5 087 005

## Description

The present invention relates to a device used to suck a highly viscous enteral nutrient such as pureed food into a syringe for injection into a gastrostoma.

Pediatric patients who require tube feeding for an extended period of time due to an eating disorder are administered nutrients by means of a gastrostomy catheter, and in recent years, highly viscous enteral nutrients such as so-called pureed food, obtained by blending natural food, have also been administered (for example, JP 2010065013 A).

Furthermore, increases in the elderly population in recent years have led to increases in the number of patients with chronic diseases such as dementia or the after-effects of a stroke, and the number of people suffering from dysphagia and aspiration pneumonia, for example, is also increasing, and thus the use of gastrostomy catheters is increasing.

Highly viscous enteral nutrients have the significant merits of enabling nutrients to be administered in a short time period, and enabling the original function of the stomach to be utilized.

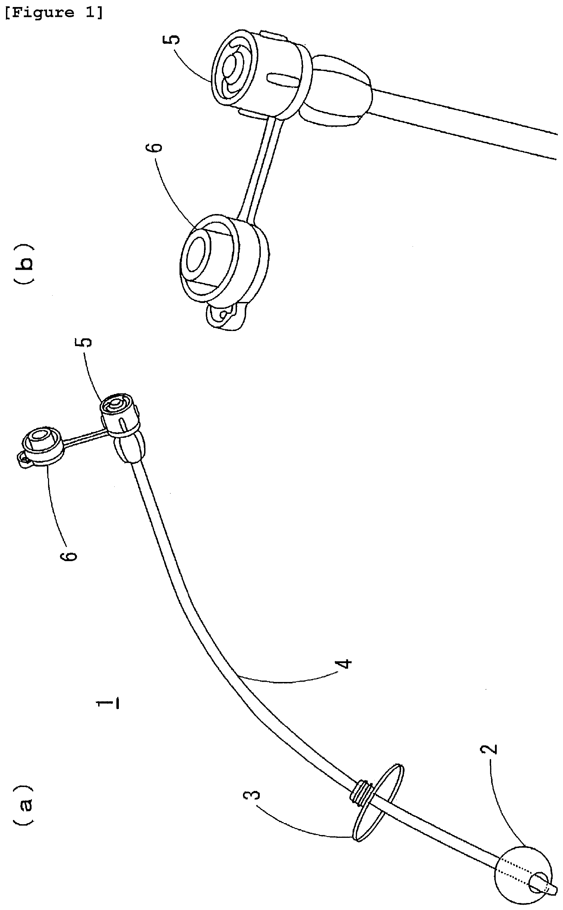

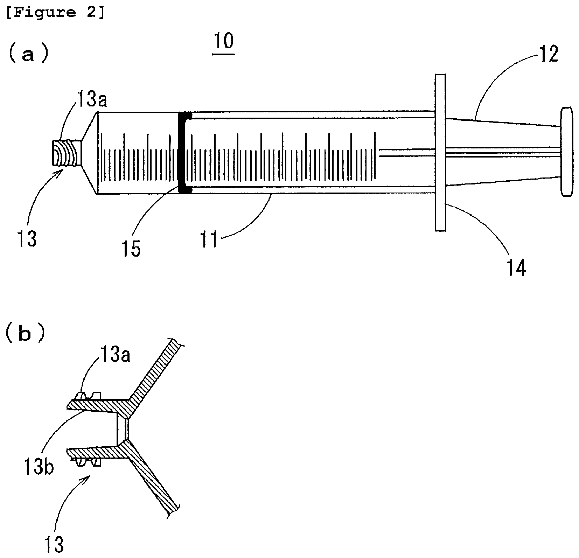

There are several types of gastrostomy catheter, and, as illustrated in Figure 1(a), one example consists of a balloon 2 which is placed inside the body, a fixing plate 3 which prevents the balloon 2 from moving toward the rear, a tube 4, a funnel 5, which is a supply port for food and the like, and a funnel stopper 6 serving as the lid thereof. Furthermore, when the enteral nutrient is to be administered, a syringe 10, illustrated in Figure 2(a), is filled with the enteral nutrient and is connected to the funnel 5 of the gastrostomy catheter 1, and the enteral nutrient in the syringe 10 is pushed out into the gastrostoma using a plunger 12.

In the syringe 10 in Figure 2(a), the plunger 12 mates with an outer tube 11. 13 is a tube tip, 14 is a flange, and 15 is a sealing material.

The shape and structure of the funnel 5 of the gastrostomy catheter 1 and the tube tip 13 of the syringe 10 are defined by an ISO standard (80369-3), with a male thread 13a being formed on the outer circumference of the tube tip 13, and a female thread formed in the funnel 5.

If the enteral nutrient has a low viscosity, the enteral nutrient can be sucked smoothly into the outer tube 11 of the syringe 10 by pulling up the plunger 12, but if the viscosity is higher than a certain level, such as with pureed food, there is a problem in that air is entrapped during sucking, and adequate filling is not achieved. Accordingly, the plunger 12 is normally pulled out from the outer tube 11 of the syringe 10, and the pureed food is scooped a number of times from the flange 14 side of the outer tube 11 using a spoon.

However, since the sealing material 15 is attached to the tip end of the plunger 12 of the syringe 10, not only does it take time and effort to pull the plunger 12 out, but there is also the problem that the pureed food adheres to the periphery of the flange 14, and must be wiped off each time to prevent dripping.

WO 2014/143130 A1 discloses a breast pump, wherein hand expression is used to express colostrum into a bowl-shaped interface, or wherein the interface contacts directly the skin of the breast while applying suction.

WO 2014/063112 A1 discloses a needle-free injection or aspiration device applied to the epidermis of a human or an animal which uses a suction cup.

It is the object of the present invention to provide an economic suction adapter with which a highly viscous enteral nutrient such as pureed food can be sucked easily into a syringe for a gastrostomy catheter.

### Means for Solving the Problem

In order to achieve this object, the suction adapter for a highly viscous enteral nutrient, according to the present invention, is configured according to the features of claim 1. That is, the adapter, being an adapter to a syringe, and being configured for sucking into the syringe a highly viscous enteral nutrient that has been arranged on a dish, and is characterized in that the suction adapter provides a suction tube, which is provided at the upper end side thereof with a connecting portion to which a tube tip of the syringe is connectable, and which is affixed to the center of a bowl-shaped suction pad, which is a resilient body, wherein a puller for pulling the suction pad away from the dish is affixed to an outer peripheral portion of the suction pad.

Further, the present invention provides a method of administering a highly viscous enteral nutrient to a syringe of a gastrostomy catheter, using a suction adapter according to claim 2.

The suction adapter is an aid for easy sucking of a highly viscous enteral nutrient, arranged on a dish, into a syringe for a gastrostomy catheter, in order to administer the highly viscous enteral nutrient to a gastrostoma, and is configured such that the suction pad is brought, from above, into close contact with the highly viscous enteral nutrient, and the highly viscous enteral nutrient is taken in by pulling up the plunger of the syringe. It should be noted that the dish is not limited to being flat, and may have a slightly concave central portion.

The suction pad is preferably a resilient body that is made of synthetic resin or synthetic rubber, for example, so as to come into close contact with the highly viscous enteral nutrient. Furthermore, the shape of the suction pad is typically a bowl shape having a circular lower surface, but is not limited thereto, and may be elliptical or polygonal. There is no particular restriction to the size. The size of the dish should be selected, as appropriate, in accordance with the properties of the highly viscous enteral nutrient.

Furthermore, the suction pad may be formed integrally with the suction tube, or as a separate member thereto, may comprise a different material, and may be mated with or screwed together with the suction tube.

The interior of the suction tube is a cavity through which the highly viscous enteral nutrient passes, and a suction inlet port is provided at the lower end thereof and a connecting portion to which the tube tip of the syringe is connected is provided at the upper end thereof. It should be noted that the upper end of the suction tube is formed with protrusions and recesses on the outer peripheral surface, or with a polygonal or elliptical cross section, to facilitate the operation of connecting to the tube tip of the syringe.

The connecting portion may have a shape provided with a female thread and a tapered guide that mates with the inner diameter of the tube tip, similar to the funnel of the gastrostomy catheter, or may be provided only with a guide, without providing a female thread, or only with a female thread, without providing a guide. It should be noted that, if a guide is not provided, a contacting surface with which the tip end of the tube tip comes into intimate contact must be provided in a coupling port to ensure hermetic attachment in the connecting portion, between the suction tube and the tube tip.

Furthermore, there is no particular restriction to the length of the suction tube. The length should be determined in consideration of ease of production and ease of attaching to and detaching from the syringe.

When the syringe has been filled with the highly viscous enteral nutrient, the syringe is detached from the suction adapter, and is connected to the funnel of the gastrostomy catheter. Since outside air flows in from the suction tube when the syringe is detached from the suction adapter, the suction pad can be easily separated from the dish by being pulled up. If it is necessary to pull the suction adapter from the dish while the syringe is still connected to the suction adapter, such as if the sucked amount is small, it is desirable to use a suction pad having a puller affixed to an outer peripheral portion thereof, as described in claim 1. Since the suction pad is strongly suction-attached to the dish, the part of the puller that is grasped should be shaped in such a way as to allow a force to be exerted easily by the hand.

### Advantages of the invention

The suction adapter for a highly viscous enteral nutrient, according to the present invention, can be easily connected to and detached from a syringe, and since air does not flow into the syringe, the highly viscous enteral nutrient can be reliably sucked into the syringe. Moreover, the structure is simple and does not require power, and can thus be used easily by anyone, and malfunctions do not occur.

### Brief explanation of the drawings

[Figure 1] illustrates an example of a gastrostomy catheter, where (a) is an overall oblique view, and (b) is an enlarged view of a funnel portion to which the tube tip of a syringe is connected.
[Figure 2] (a) is a front view illustrating the entire syringe, and (b) is an enlarged cross-sectional view of the tube tip.
[Figure 3] (a) is an overall oblique view illustrating an embodiment of the suction adapter, and (b) is a cross-sectional front view.
[Figure 4] (a) is similarly an enlarged cross-sectional view of the syringe and the connecting portion of the suction adapter, and a cross-sectional view illustrating a connected state. (b) and (c) are cross-sectional views illustrating the connecting portions of other suction adapters, and the connected state.
[Figure 5] is an oblique view illustrating other exemplary embodiments of the suction pad.
[Figure 6] is an oblique view illustrating exemplary embodiments of the puller.
[Figure 7] is a cross-sectional front view illustrating the usage state of the suction adapter.

### Best Mode for Carrying Out the Invention

An embodiment of the suction adapter for a highly viscous enteral nutrient, according to the present invention, will be described on the basis of the accompanying drawings.

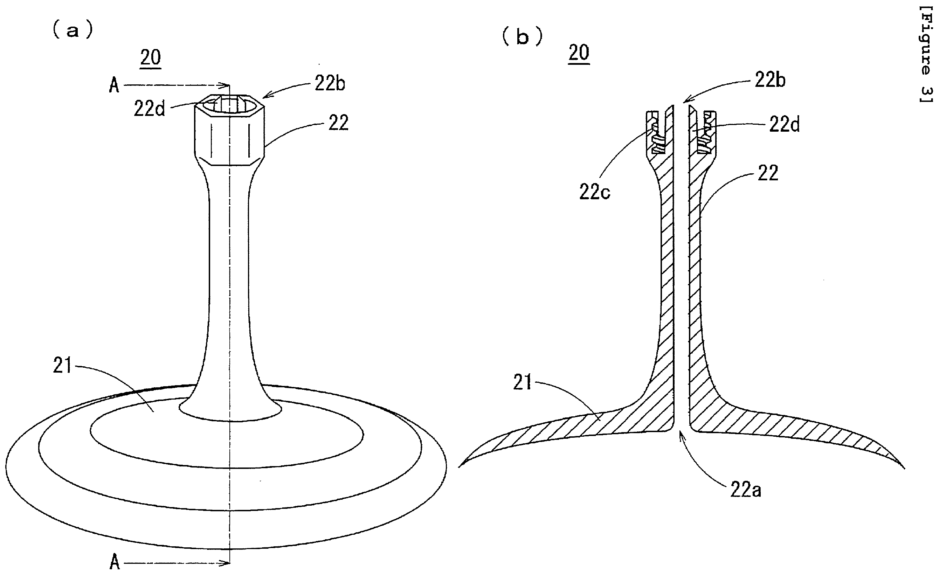

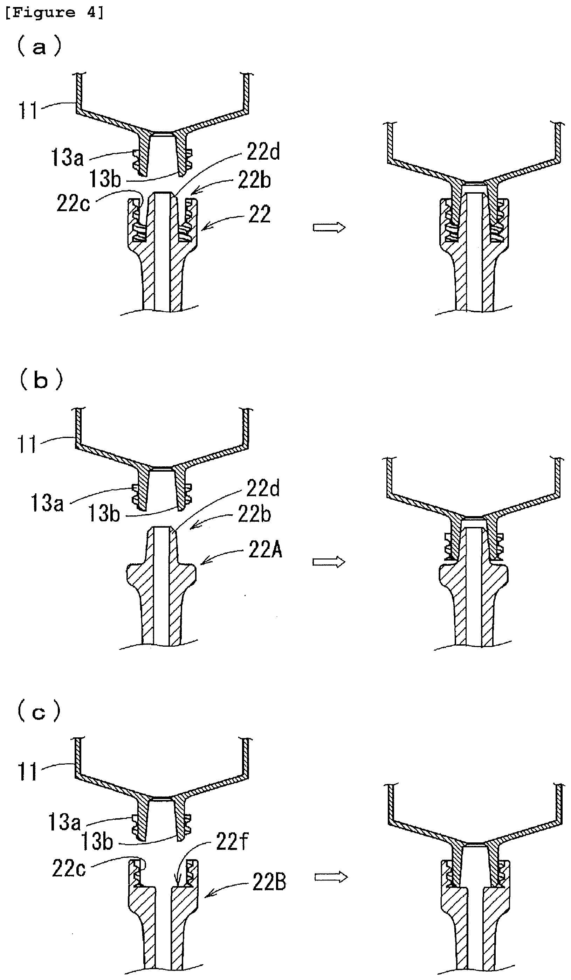

Figure 3 (a) and (b) are an oblique view and a cross-sectional front view illustrating an embodiment of a suction adapter 20 according to the present invention. Furthermore, Figure 4 (a), (b), and (c) are enlarged views of a connecting portion of the suction adapter 20 and the syringe 10, and a cross-sectional view illustrating the connected state.

The suction adapter 20 is integrally molded using a synthetic resin (such as polyvinyl chloride), a suction pad 21 comprising a bowl-shaped resilient body is formed at the tip end thereof, and a suction tube 22 is provided upright in a central portion thereof. A communicating hole is formed in a central portion of the suction tube 22, a suction inlet port 22a is formed on the suction pad 21 side, and a connecting portion 22b to which the tube tip 13 of the syringe 10 is connected is formed on the other end side.

As illustrated in Figure 4(a), a female thread 22c with which the male thread 13a of the tube tip 13 of the syringe 10 meshes is formed on the upper end side of the connecting portion 22b, and a guide 22d which engages with an inner diameter 13b of the tube tip 13 of the syringe 10 is formed on the inside thereof. The guide 22d is formed with a tapered shape which has a small diameter on the connecting portion 22b side, and which gradually becomes larger with increasing distance toward the rear, and a hole which has the same diameter as far as the suction inlet port 22a is formed in a central portion of the guide 22d.

The method for using the suction adapter 20 configured as described above will next be described.

For example, if pureed food, which is a highly viscous enteral nutrient, is to be administered to a gastrostoma, the pureed food is first poured directly from a blender onto a dish 30, or is removed therefrom using a spoon or the like.

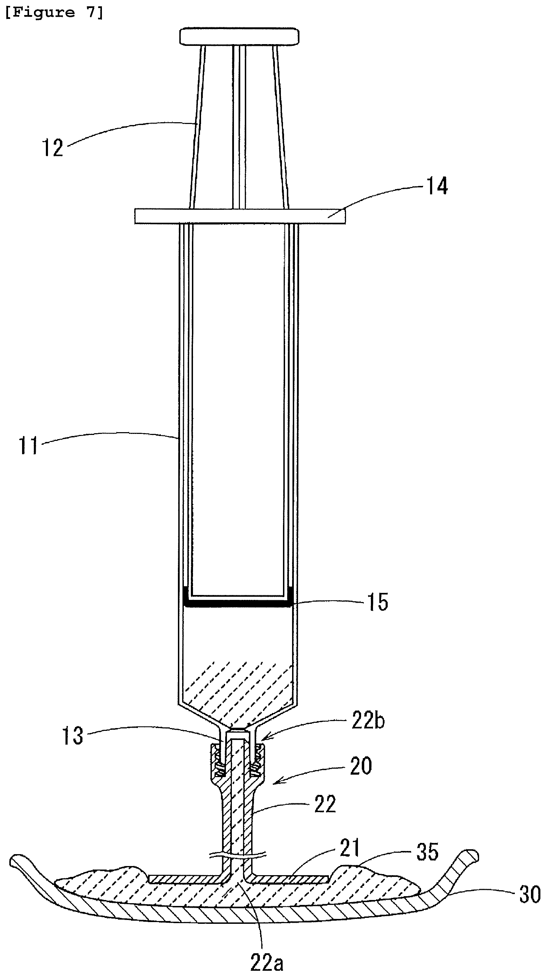

Next, as illustrated in Figure 7, the tube tip 13 of the syringe 10 is connected to the connecting portion 22b, which is the upper end of the suction tube 22 of the suction adapter 20, but first the plunger 12 of the syringe 10 is pushed in as far as the bottom of the outer tube 11, and the male thread 13a of the tube tip 13 is screwed into the female thread 22c of the connecting portion 22b of the suction tube 22. As a result, the inner diameter 13b of the tube tip 13 mates with and comes into close contact with the outer diameter of the guide 22d of the suction tube 22.

Then, with the suction pad 21 on the lower side, and while being held substantially vertical, the suction pad is pressed lightly against the pureed food 35 on the dish 30, from the upper surface thereof.

If the plunger 12 of the syringe 10 is pulled up while in this state, the pureed food 35 is sucked up and sucked into a space from where the plunger 12 of the syringe 10 has been pulled up.

Next, the connecting portion 22b of the suction adapter 20 is detached from the tube tip 13 of the syringe 10, the tube tip 13 of the syringe 10 is connected, by being screwed, to the funnel 5 of the gastrostomy catheter 1 illustrated in Figure 1, and the plunger 12 is pushed in to deliver the pureed food 35 to the gastrostoma.

It should be noted that, since outside air flows in from the suction tube 22, the suction adapter 20 from which the syringe 10 has been removed can easily be detached from the dish 30.

In order to continue delivering the pureed food 35 to the gastrostoma, the syringe 10 should be removed from the funnel 5 and replaced on the suction adapter 20, and the same operation as described hereinabove should be repeated.

Other embodiments of the suction tube 22 of the suction adapter 20 will next be described on the basis of Figure 4(b) and Figure 4(c).

The suction tube 22A in Figure 4(b) comprises the suction tube 22 described hereinabove without the female thread 22c of the connecting portion 22b, and is connected to the syringe 10 by means of mating between the inner diameter 13b of the tube tip 13 and the guide 22d of the suction tube 22. The structure of the suction tube 22 is simpler than that described hereinabove, and workability when connecting to the syringe 10 is also better since the screwing operation described hereinabove is not required.

The suction tube 22B in Figure 4(c) comprises the suction tube 22 described hereinabove without the guide 22d of the connecting portion 22b, and is connected to the syringe 10 by means of mating between the male thread 13a of the tube tip 13 and the female thread 22c of the suction tube 22B.

The suction tube 22B has a simple structure, but when used, the male thread 13a must be screwed in such that the tip end of the tube tip 13 of the syringe 10 comes into close contact with a contacting surface 22f of a coupling port 22.

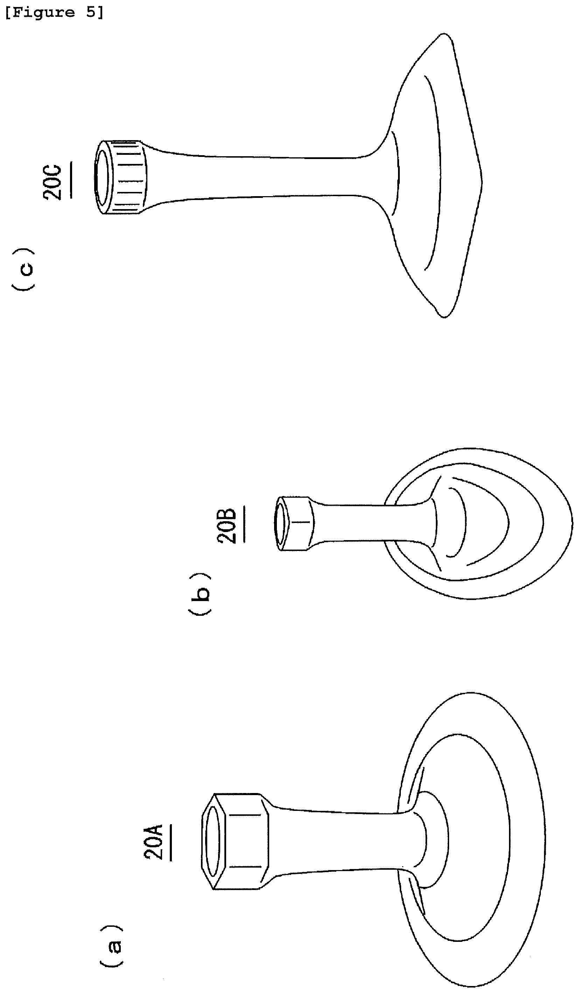

Figure 5 illustrates various embodiments of the suction adapter, where suction adapter 20A represents a case in which the suction tube 22 and the suction pad 21 have a large diameter, and suction adapter 20B represents a case in which the size is small and the lower surface of the suction pad 21 is elliptical. Furthermore, suction adapter 20C represents a case in which the suction tube 22 is long, and the lower surface of the suction pad 21 is polygonal.

A decision regarding which to use should be made in accordance with the properties of the highly viscous enteral nutrient and the size and shape of the dish 30 being used.

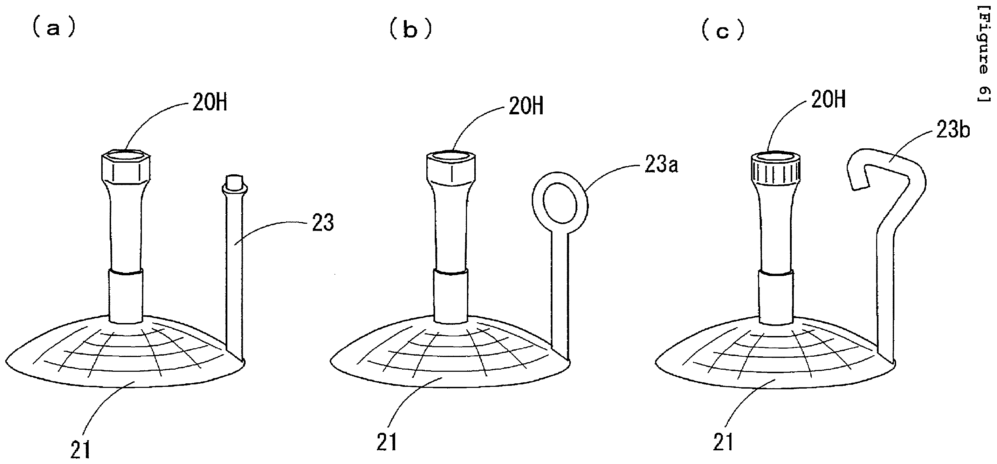

Embodiments of claim 2 will next be described on the basis of Figure 6.

In a state in which the syringe 10 is coupled to the suction adapter 20 and the highly viscous enteral nutrient has been sucked in, the suction pad 21 is suction-attached to the dish 30, and the suction pad 21 cannot be pulled away from the dish 30. In the suction adapters illustrated in Figure 6, a puller 23 is affixed to an outer peripheral portion of the suction pad 21 to enable the suction pad to be pulled away from the dish 30 in such a case.

Various pullers 23 are conceivable, as illustrated in Figure 6(a) to (c). The puller in (a) is in the shape of a rod provided upright on an outer peripheral portion of the suction pad 21, and with a convex grasping portion on which fingers can be placed formed at the upper end, in (b), a ring-shaped handle is attached to the upper end, and in (c), the upper end has a hook shape.

If a suction adapter 20H provided with such a puller is employed, after a prescribed amount of the prepared pureed food 35 has been sucked into the syringe 10, for example, the suction pad 21 can easily be pulled away from the dish 30 by pulling the puller 23 of the suction adapter 20H, and a separately prepared pureed food, or the remaining pureed food, can be additionally sucked into the syringe 10.

### Explanation of the reference numbers

- 1: Gastrostomy catheter
- 2: Balloon
- 3: Fixing plate
- 4: Tube
- 5: Funnel
- 6: Funnel stopper
- 10: Syringe
- 11: Outer tube
- 12: Plunger
- 13: Tube tip
- 13a: Male thread
- 13b: Inner diameter
- 14: Flange
- 15: Sealing material
- 20: Suction adapter
- 20A, 20B, 20C, 20H: Suction adapter
- 21: Suction pad
- 22: Suction tube
- 22A,: 22B Suction tube
- 22a: Suction inlet port
- 22b: Connecting portion
- 22c: Female thread
- 22d: Guide
- 22f: Contacting surface
- 23, 23a, 23b: Puller
- 30: Dish
- 35: Pureed food

## Claims

1. A suction adapter for a highly viscous enteral nutrient (35), being an adapter (20, 20A, 20B, 20C, 20 H) to a syringe (10), and being configured for sucking into the syringe (10) the highly viscous enteral nutrient (35) that has been arranged on a dish (30), **characterized in that** the suction adapter (20, 20A, 20B, 20C, 20 H) provides a suction tube (22, 22A, 22B), which is provided at the upper end side thereof with a connecting portion (22b) to which a tube tip (13) of the syringe (10) is connectable, and which is affixed to the center of a bowl-shaped suction pad (21), which is a resilient body,
**characterized in that** a puller (23, 23a, 23b) for pulling the suction pad (21) away from the dish (30) is affixed to an outer peripheral portion of the suction pad (21).

2. Method of administering a highly viscous enteral nutrient (35) to a syringe (10) of a gastrostomy catheter (1) using a suction adapter (20, 20A, 20B, 20C, 20 H) providing a suction tube (22, 22A, 22B), which is provided at the upper end side thereof with a connecting portion (22b) to which a tube tip (13) of the syringe (10) is connectable, and which is affixed to the center of a bowl-shaped suction pad (21), which is a resilient body, the method comprising:
connecting the suction adapter (20, 20A, 20B, 20C, 20 H) to the syringe (10);
bringing the suction pad (21) into contact with the highly viscous enteral nutrient (35) that has been arranged on a dish (30);
suctioning the highly viscous enteral nutrient (35) into the syringe (10).

## Patentansprüche

1. Saugadapter für eine hochviskose enterale Nahrung (35), ausgebildet als Adapter (20, 20A, 20B, 20C, 20H) für eine Spritze (10) und dazu bestimmt, die auf einem Gefäß (30) angeordnete hochviskose enterale Nahrung (35) in die Spritze (10) einzusaugen, **dadurch gekennzeichnet, dass** der Saugadapter (20, 20A, 20B, 20C, 20H) ein Saugrohr (22, 22A, 22B) aufweist, das an seinem oberen Ende eine Anschlussstelle (22b) besitzt, an die eine Rohrspitze (13) der Spritze (10) anschließbar ist, und das in der Mitte eines schalenförmigen Saugpads (21), welches ein elastischer Körper ist, befestigt ist,
wobei ein Ziehgriff (23, 23a, 23b) zum Abziehen des Saugpads (21) von dem Gefäß (30) an einem äußeren Randbereich des Saugpads (21) befestigt ist.

2. Verfahren zur Verabreichung einer hochviskosen enteralen Nahrung (35) an eine Spritze (10) eines Gastrostomie-Katheters (1) unter Verwendung eines Saugadapters (20, 20A, 20B, 20C, 20H), der ein Saugrohr (22, 22A, 22B) aufweist, das an seinem oberen Ende einen Verbindungsbereich (22b) besitzt, an dem eine Rohrspitze (13) der Spritze (10) anschließbar ist, und das in der Mitte eines schalenförmigen Saugpads (21), welches ein elastischer Körper ist, befestigt ist, wobei das Verfahren umfasst:
das Verbinden des Saugadapters (20, 20A, 20B, 20C, 20H) mit der Spritze (10);
die Kontaktierung des Saugpads (21) mit der hochviskosen enteralen Nahrung (35), die auf einem Gefäß (30) angeordnet ist;
und das Ansaugen der hochviskosen enteralen Nahrung (35) in die Spritze (10).

## Revendications

1. Adaptateur d'aspiration pour un nutriment entéral hautement visqueux (35), constituant un adaptateur (20, 20A, 20B, 20C, 20H) pour une seringue (10) et étant configuré pour aspirer dans la seringue (10) le nutriment entéral hautement visqueux (35) disposé sur un récipient (30), **caractérisé en ce que** l'adaptateur d'aspiration (20, 20A, 20B, 20C, 20H) comporte un tube d'aspiration (22, 22A, 22B) pourvu à son extrémité supérieure d'une partie de raccordement (22b) à laquelle une embout de tube (13) de la seringue (10) peut être connectée, et qui est fixé au centre d'une ventouse (21) en forme de coupe qui est un corps élastique,
et qu'un élément de traction (23, 23a, 23b) destiné à détacher la ventouse (21) du récipient (30) est fixé à une partie périphérique extérieure de la ventouse (21).

2. Procédé d'administration d'un nutriment entéral hautement visqueux (35) à une seringue (10) d'un cathéter de gastrostomie (1) en utilisant un adaptateur d'aspiration (20, 20A, 20B, 20C, 20H) qui comporte un tube d'aspiration (22, 22A, 22B) pourvu à son extrémité supérieure d'une partie de raccordement (22b) à laquelle une embout de tube (13) de la seringue (10) peut être connectée, et fixé au centre d'une ventouse (21) en forme de coupe qui est un corps élastique, le procédé comprenant:
la connexion de l'adaptateur d'aspiration (20, 20A, 20B, 20C, 20H) à la seringue (10);
la mise en contact de la ventouse (21) avec le nutriment entéral hautement visqueux (35) disposé sur un récipient (30);
et l'aspiration du nutriment entéral hautement visqueux (35) dans la seringue (10).
